Europäisches Patentamt

European Patent Office

Office européen des brevets

(19)

(11) Veröffentlichungsnummer: **0 336 884**

**A1**

## (12) EUROPÄISCHE PATENTANMELDUNG

(21) Anmeldenummer: **89810138.1**

(22) Anmeldetag: **22.02.89**

(51) Int. Cl.⁴: $A\ 61\ F\ 2/34$

(30) Priorität: **29.03.88 CH 1182/88**

(43) Veröffentlichungstag der Anmeldung:
**11.10.89 Patentblatt 89/41**

(84) Benannte Vertragsstaaten: **AT DE FR GB IT**

(71) Anmelder: **GEBRÜDER SULZER
AKTIENGESELLSCHAFT
Zürcherstrasse 9
CH-8401 Winterthur (CH)**

(72) Erfinder: **Willert, Hans-Georg, Prof. Dr.-med.
Schlegelweg 9
D-3400 Göttingen (DE)**

**Bürgi, Maja
Chaennerwisstrasse 5
CH-8352 Räterschen (CH)**

(54) **Armierung für ein Knochenzementbett.**

(57) Eine aus Wellenzügen (1) gebildete Armierung für ein Knochenzementbett (8) ist mit nach aussen vorstehenden, dornartigen Fixierungselementen (5, 6) versehen. Diese dienen dazu, die Armierung an dem Knochen (7) zu fixieren, ehe der Knochenzement (8) eingebracht wird.

Fig 2

EP 0 336 884 A1

## Beschreibung

**Gebrüder Sulzer, Aktiengesellschaft, Winterthur/Schweiz**

Armierung für ein Knochenzementbett

Die Erfindung betrifft eine aus einem Drahtgitter bestehende Armierung für ein eine Endoprothese umgebendes Knochenzementbett, welche Armierung auf der Aussen-Oberfläche der Prothese abnehmbar aufliegt und aus mindestens zwei Wellenzügen gebildet wird, die sich mindestens annähernd unter einem rechten Winkel schneiden.

Eine derartige Armierung ist beispielsweise für den Verankerungsschaft einer Femurkopfprothese aus der US-PS 4,064,567 bekannt. Diese bekannte Armierung ist strumpfartig über den Schaft gezogen und wird bei der Implantation zusammen mit diesem in den zuvor in den Knochen eingebrachten Knochenzement eingepresst. Dabei wird der schon auspolimerisierende Knochenzement von den eindringenden Drähten in einzelne Sektoren zerschnitten, die zumindest nicht wieder vollständig zu einer geschlossenen Masse "zusammenfliessen", sondern zwischen denen mindestens teilweise "Trennwände" verbleiben. Man ist daher heute bestrebt, die Armierung vor dem Knochenzement in den Knochen einzubringen, so dass der beim Eindringen noch relativ flüssige Knochenzement die Armierung allseitig umfliesst und dann zu einem geschlossenen und, abgesehen von der Armierung, homogenen Zementbett polimerisiert.

Besonders für Armierungen von im Becken zu verankernden Hüftgelenkspfannen haben sich bei der Fixierung vor dem Einbringen des Knochenzementes Schwierigkeiten ergeben, da sich die Haftung der Armierung im Knochen bei einfachem elastischen Einklemmen als ungenügend erwiesen hat. Es ist deshalb Aufgabe der Erfindung eine Armierung zu schaffen, die im operativ geschaffenen Knochenhohlraum fixiert werden kann, bevor der Knochenzement und das Implantat eingebracht sind.

Erfindungsgemäss wird diese Aufgabe dadurch gelöst, dass an einzelnen Punkten der Wellenzüge in den Knochen eindringende, dornartige Fixierungselemente vorgesehen sind.

Die dornenartigen Ansätze lassen sich leicht in das im allgemeinen spongiöse Knochengewebe eindrücken und gewährleisten ein Haften der Armierung am Knochen bis diese durch den einfliessenden und aushärtenden Knochenzement endgültig fixiert wird.

Als Armierung für das Zementbett zur Fixierung einer halbkugelförmigen Hüftgelenksschale hat sich eine Konstruktion bewährt, bei der die Wellenzüge in Meridianrichtung der Pfanne verlaufen und durch sich entlang Breitenkreisen erstreckende Stützringe zu einem weitmaschigen Gitter ergänzt sind; einer der dornenartigen Ansätze kann dabei mit Vorteil am Pol der Halbkugel angeordnet sein, während andere, die auch als Agraffen ausgebildet sein können, zweckmässigerweise an Schnittstellen von Wellenzügen und Stützringen angeordnet sein können.

Um ein gleichmässig ausreichend dickes Zementbett um das Implantat herum sicherzustellen, ist es schliesslich vorteilhaft, wenn der vertikale Scheitelabstand der Wellenzüge 2 - 4 mm beträgt.

Bevorzugte Materialien für die Armierung sind Titan oder Titanlegierungen, sowie rostfreie Stähle.

Im folgenden wird die Erfindung anhand eines Ausführungsbeispiels im Zusammenhang mit der Zeichnung näher erläutert.

Fig. 1 zeigt schematisch einen Meridianschnitt durch eine Armierung für eine Hüftgelenkspfanne;

Fig. 2 gibt einen Schnitt durch eine unter Verwendung der Armierung nach Fig. 1 mit Hilfe eines Knochenzementbettes fixierte Hüftgelenkspfanne wieder.

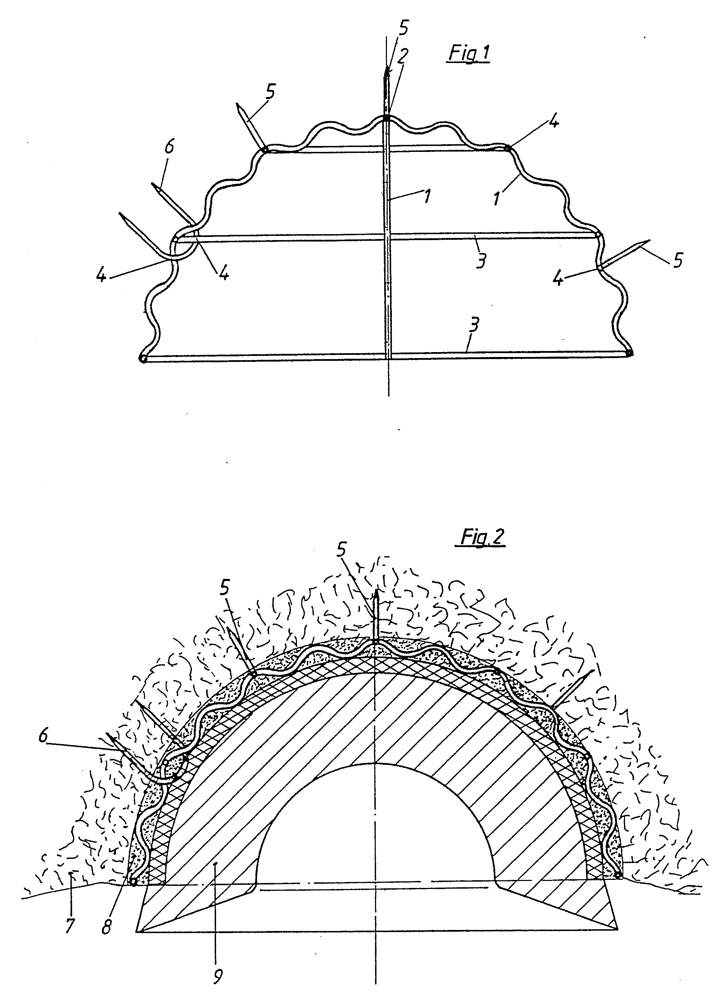

Die Armierung von Fig. 1 besteht aus zwei mindestens nahezu senkrecht zueinander angeordneten Wellenzügen 1, deren "Amplitudenhöhe" einige Millimeter beträgt. Die Wellenzüge 1 verlaufen in Meridianebenen einer fiktiven Halbkugel und sind am Pol 2 durch eine Löt- oder Schweissverbindung miteinander verbunden. Die Stabilität der Armierung gewährleisten Stützringe 3, die auf verschiedenen "geographischen Breiten" im Innern der Wellenzüge 1 verlaufen. An ihnen sind die Wellenzüge 1 ebenfalls durch Löt-oder Schweissverbindungen 4 befestigt.

Erfindungsgemäss erstrecken sich aus der "Oberfläche" der Wellenzüge 1 im wesentlichen radial nach aussen dornartige Fixierungselemente 5, die, wiederum durch Löten oder Schweissen, an die Wellenzüge 1 angeheftet sind. Die Elemete 5, die bevorzugt an Schnittpunkten der Wellenzüge 1 untereinander oder an solchen mit den Stützringen 4 angeordnet sind, müssen - entgegen der Darstellung - nicht in einer Ebene liegen, sondern können willkürlich auf der Oberfläche der Armierung räumlich verteilt sein.

Weiterhin können die dornartigen Fixierungselemente auch als Agraffen 6 ausgebildet sein, die entweder an Wellenzügen 1 oder Stützringen 3 angeheftet sind (Löt- oder Schweissstellen 4) oder die Armierung von innen nach aussen lose umgreifen.

Wie bereits beschrieben, wird die Armierung während der Implantation mit den Elementen 5 und/oder 6 zunächst an die Spongiosa 7 (Fig. 2) angeheftet, ehe sie im Knochenzement 8 eingebettet wird. In diesen wird schliesslich die Pfanne 9 eingepresst. Nach dem Aushärten des Zementes 8 ist die Pfanne 9 auf diese Weise im Knochen 7 verankert, wobei infolge der Armierung ein über die ganze Oberfläche gleichmässig dickes Knochenzementbett 8 gebildet worden ist.

## Patentansprüche

1. Aus einem Drahtgitter bestehende Armierung für ein eine Endoprothese umgebendes Knochenzementbett, welche Armierung auf der Aussen-Oberfläche der Prothese abnehmbar aufliegt und aus mindestens zwei Wellenzügen gebildet wird,die sich mindestens annähernd unter einem rechten Winkel schneiden, **dadurch gekennzeichnet, dass** an einzelnen Punkten der Wellenzüge (1) in den Knochen (7) eindringende, dornartige Fixierungselemente (5,6) vorgesehen sind.

2. Armierung nach Anspruch 1 für das Knochenzementbett einer mindestens annähernd halbkugelförmigen, künstlichen Hüftgelenkspfanne, **dadurch gekennzeichnet dass** die Wellenzüge (1) in Meridianrichtung der Pfanne (9) verlaufen und durch sich entlang Breitenkreisen erstreckende Stützringe (3) zu einem weitmaschigen Gitter ergänzt sind.

3. Armierung nach Anspruch 2, **dadurch gekennzeichnet, dass** eines (5) der Fixierungselemente (5,6) am Pol (2) der Halbkugel angeordnet ist.

4.Armierung nach Anspruch 2 oder 3, **dadurch gekennzeichnet, dass** agraffenartige Fixierungselemente (6) an Schnittstellen von Wellenzügen (1) und Stützringen (3) vorgesehen sind.

5. Armierung nach einem der Ansprüche 1 bis 4, **dadurch gekennzeichnet, dass** der vertikale Scheitelabstand der Wellenzüge (1) 2 - 4 mm beträgt.

EP 0 336 884 A1

Fig 1

Fig 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 412 304 (THE SAMPSON CORP.) <br> * Figuren 1,5 * <br> ----- | 1 | A 61 F 2/34 |

RECHERCHIERTE SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 14-07-1989 | ARGENTINI A. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes
   Dokument